# EUROPEAN PATENT SPECIFICATION

(11) **EP 2 124 935 B1**
(45) Date of publication and mention of the grant of the patent: **25.04.2012**
(21) Application number: 08708944.7
(22) Date of filing: 13.02.2008
(51) Int. Cl.: A61K 31/403, A61K 31/427, A61K 31/496, A61K 31/546, A61P 31/00, A61K 31/43, A61K 31/545

(54) **ANTIBACTERIAL COMBINATION AND ITS USE**
ANTIBAKTERIELLE KOMBINATION UND IHRE VERWENDUNG
COMBINAISON ANTIBACTÉRIENNE ET SON UTILISATION

(30) Priority: 13.02.2007 EP 07102241
(43) Date of publication of application: 02.12.2009
(73) Proprietor: LEK Pharmaceuticals d.d., 1526 Ljubljana (SI)
(72) Inventor: PREZELJ, Andrej, 1000 Ljubljana (SI); URLEB, Uros, 1000 Ljubljana (SI); SOLMAJER, Tomaz, 1000 Ljubljana (SI)
(74) Representative: Wichmann, Hendrik
(86) International application number: PCT/EP2008/051724
(87) International publication number: WO 2008/098955

(56) References cited:
- WO-A-98/27094
- VILAR M ET AL: "Kinetic study of two novel enantiomeric tricyclic [beta]-lactams which efficiently inactivate class C [beta]-lactamases" ANTIMICROBIAL AGENTS AND CHEMOTHERAPY 2001 UNITED STATES, vol. 45, no. 8, 2001, pages 2215-2223, XP008080169 ISSN: 0066-4804
- COPAR, ANTON ET AL: "Design, synthesis and bioactivity evaluation of tribactam .beta. lactamase inhibitors" BIOORGANIC & MEDICINAL CHEMISTRY LETTERS , 12(6), 971-975 CODEN: BMCLE8; ISSN: 0960-894X, 2002, XP002478137
- GOODMAN G A ET AL: "GOODMAN and GILMAN'S The Pharmacological Basis of Therapeutics , (The cephalosporins)" 2001, GOODMAN AND GILMAN'S THE PHARMACOLOGICAL BASIS OF THERAPEUTICS, NEW YORK : MCGRAW-HILL, US, PAGE(S) 1206-1213 , XP002438160 ISBN: 0-07-135469-7 the whole document
- PETRI W A ED - HARDMAN J G ET AL: "GOODMAN and GILMAN'S The Pharmacological Basis of Therapeutics , (The penicillins), Chapter 45 - Antimicrobial Agents (continued) Penicillins, Cephalosporins, and Other beta-Lactam Antibiotics" 1 January 2001 (2001-01-01), GOODMAN AND GILMAN'S THE PHARMACOLOGICAL BASIS OF THERAPEUTICS, NEW YORK : MCGRAW-HILL, US, PAGE(S) 1189 - 1206 , XP002451319 ISBN: 978-0-07-135469-1 the whole document
- PETRI W A ED - HARDMAN J G ET AL: "GOODMAN and GILMAN'S The Pharmacological Basis of Therapeutics , (Other beta-lactam antibiotics), Chapter 45 - Antimicrobial Agents: Penicillins, Cephalosporins, and Other beta-Lactam Antibiotics" 1 January 2001 (2001-01-01), GOODMAN AND GILMAN'S THE PHARMACOLOGICAL BASIS OF THERAPEUTICS, NEW YORK : MCGRAW-HILL, US, PAGE(S) 1206 - 1213 , XP002451320 ISBN: 978-0-07-135469-1 the whole document

## Description

### FIELD OF INVENTION

The present invention relates to the field of new antimicrobial drugs, especially to a pharmaceutical composition comprising ethylidene derivatives of tricyclic carbapenem or pharmaceutically acceptable salts thereof in combination with an antibiotic, especially with an antibiotic that is susceptible to degradation by β-lactamase and the use of a respective pharmaceutical composition for the treatment of an infection in humans or animals caused by a bacterium, as defined in the claims.

### BACKGROUND OF THE INVENTION

The dramatic increase in bacterial antibiotic resistance has become one of the most important global threats to modern health care. Although bacteria have developed several strategies for escaping the lethal action of antibiotics the most common and often the most efficient mechanism of bacterial resistance to β-lactam antibiotics is the expression of β-lactamases. The bacterial β-lactamase enzymes hydrolyze antibiotics of β-lactam family, e.g. penicillins, cephalosporins, monobactams, carbapenems, to inactive products by hydrolyzing the β-lactam bond. This type of resistance can be transferred horizontally by plasmids that are capable of rapidly spreading the resistance, not only to other members of the same strain, but even to other species. The diversity of β**-**lactamases is a most critical aspect of antimicrobial therapy. Currently the β-lactamase super-family has more than 550 members, many of which differ only by a single amino acid. Based on amino-acid sequence similarities, β-lactamases have been broadly grouped into four molecular Classes, A, B, C and D. [Bush K; et al; Antimicrob. Agents Chemother. 1995, 39 (6): 1211-1233; Thomson KS; et al; Microbes and Infections 2000, 2: 1225-1235].

Important strategy for overcoming worldwide emergence of bacterial resistance is administration of β-lactamase inhibitors, which lack antibiotic activity themselves and are thus administered together with an antibiotic. Commercially available β-lactamase inhibitors such as potassium clavulanate, sulbactam and tazobactam have been successfully used in synergistic mixtures against bacteria producing the ubiquitous and prevalent TEM-1 and SHV-1 Class A β-lactamases. However, little or no activity against Class C and B enzymes was observed. In addition, bacterial susceptibility to such combinations has recently been challenged by the spontaneous appearance of new β-lactamases of the TEM family, which are resistant to the mechanism-based inactivators in the market. Any organism with an inducible AmpC β-lactamase (Class C) can segregate derepressed mutants, and any TEM, SHV or CTX-M producer can segregate ESBL (extended spectrum β-lactamase) variants. [Livermore, DM. J. Antimicrob. Chemother. 1998, 41 (D), 25-41; Livermore, DM. Clinical Microbiology Reviews 1995, 8 (4), 557-584; Helfand MS; et al; Curr. Opin. Pharmacol. 2005, 5: 452-458].

Attempts to address the above mentioned problems through the development of β-lactamase inhibitors had only limited success in the past. However, a detailed knowledge of binding mechanism and interactions of known β-lactam should facilitate the design of novel β-lactam that will bypass this defense mechanism.

Alkylidene penems and 2β-substituted penam sulphones, oxapenems, cephalosporin-derived compounds, cyclic acyl phosphonates, and non-β-lactam compounds are currently under investigation as potential β-lactamase inhibitors, but their Clinical applications are not yet available [Buynak JD. Curr. Med. Chem. 2004, 11, 1951-1964; Bonnefoy A et al, J. Am. Chem. Soc. 2004, 54, 410-417; Weiss WJ et al; Antimicrob. Agents Chemother. 2004, 48, 4589-4596, Phillips OA at al; J. Antibiot. 1997, 50, 350-356; Jamieson CE et al; Antimicrob. Agents Chemother. 2003, 47, 1652-1657].

Therefore, one subject of the present invention is the improvement of the stability of enzyme-inhibitor complexes and the design of efficient compounds (high acylation and low deacylation rates) that are resistant to inactivation by β-lactamases. Another subject of the present invention is to provide new pharmaceutical combinations that show a potency and spectrum for the most prevalent Clinically relevant resistant strains.

Surprisingly it has been found that ethylidene derivatives of tricyclic carbapenems of Formula (I) and also salts, esters or amides thereof, which are described in WO 98/27094 used as β-lactamase inhibitors are suitable to combat emerging bacterial resistance in Class A, Class C and Class D β-lactamases and provide a vital addition to the hospital antibiotic armory. It also has been found that the combination between these second-generation β-lactamase inhibitors and a β-lactam antibiotic provides pharmaceutical compositions that are suitable for the treatment of an infection caused by a bacterium.

### SUMMARY OF THE INVENTION

Thus, the description reports the use of ethylidene derivatives of tricyclic carbapenems of Formula (I) and also salts, esters or amides thereof, which are described in WO98/27094. Theses compounds, which are structurally unrelated to the natural product and semi-synthetic β-lactamase inhibitors presently available, significantly increase the efficacy of β-lactam antibiotics against several bacteria dramatically and therefore can be used as broad spectrum β-lactamase inhibitors, especially β-lactamase inhibitors of Class A, C and D.

The invention is directed towards a pharmaceutical composition comprising a compound mentioned above and an antibiotic, especially a β-lactam antibiotic, as defined in the claims. These pharmaceutical compositions may additionally comprise a pharmaceutically acceptable carrier and/or a pharmaceutically acceptable excipient. These pharmaceutical compositions are particularly suitable for treatment of an infection in humans or animals caused by a bacterium, especially a bacterium that produces a significant amount of β-lactamase.

Methods for inhibiting bacterial growth are also described. Methods described herein comprise administering a β**-**lactamase inhibitor of the invention in combination with antibiotics, preferably β-lactam antibiotics to a bacterial cell culture, or to a bacterially infected cell culture, tissue, or organism.

### DETAILED DESCRIPTION OF THE INVENTION

Ethylidene derivatives of tricyclic carbapenem of Formula (I) or a salt, ester or amide derivative thereof both inhibit enzymatic activity of β-lactamase *in vitro* and enhance the potency of antibiotic agents in bacterial cell culture and are useful for the treatment of infections in humans and animals. In contrast to other known carbapenem antibiotics the ethylidene derivative of tricyclic carbapenem of Formula (I) possess no significant intrinsic antibiotic activity.

Thus, described are ethylidene derivatives of tricyclic carbapenem of Formula (I) 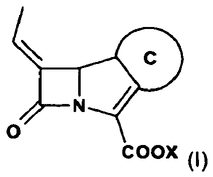 or a salt, ester or amid derivate thereof , wherein the ring marked C which is fused to the basic carbapenem nucleus in 3 and 4 position, is a five-, six- or seven-membered ring whereat
i) one or more carbon atoms in the ring marked C may be mono or disubstituted with substituents which may be the same or different and may mean:
   a) a hydrogen atom,
   b) a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl) and each chain member may be mono or disubstituted with substituents such as halo (such as fluoromethyl, trifluoromethyl, 2-chloroethyl), hydroxy (such as hydroxymethyl, 2-hydroxyethyl), (C₁-C₄)-alkyloxy (such as methoxymethyl, 2-methoxyethyl), mercapto and (C₁-C₄)-alkylmercapto (such as mercaptomethyl, 2-methylmercaptoethyl), (C₁-C₄)-alkanesulfonyl (such as methanesulfonylmethyl), amino, (C₁-C₄)alkylamino and di(C₁-C₄)-alkylamino (such as 2-aminoethyl, 2-methylaminoethyl, 2-dimethylaminoethyl), alkyleneamino (such as 2-(1-piperidinyl)ethyl, 1-pyrrolidinylmethyl), guanidino (such as guanidinomethyl), unsubstituted N¹-mono, N³-mono, N¹,N³-di and N³,N³-di-(C₁-C₄)-formamidino (such as iminomethylaminomethyl, 2-(dimethylaminomethyleneamino)ethyl), aromatic or heteroaromatic five- or six-membered ring (such as phenyl, furyl, 2-pyridyl), (C₁-C₄)-alkyloxycarbonyl (such as carbethoxymethyl), cyano (such as 2-cyanoethyl), oxo (such as acetyl, propionyl, 2-oxopropyl),
   c) an unsaturated alkyl chain with 1 to 20 carbon atoms and the unsaturated alkyl chain may be straight with double bonds or triple bonds (such as vinyl, propenyl, allyl, ethinyl, propargyl) or branched in any position with double bonds or triple bonds (such as 2-propenyl) and each chain member may be mono or disubstituted with substituents such as halo, hydroxy, (C₁-C₄)-alkyloxy, thio and (C₁-C₄)-alkylthio, (C₁-C₄)-alkanesulfonyl, amino, (C₁-C₄)-alkylamino and di-(C₁-C₄)-alkylamino, aromatic or heteroaromatic five- or six-membered ring (such as phenyl, furyl, 2-pyridyl), (C₁-C₄)-alkyloxycarbonyl, cyano, oxo,
   d) a saturated or partly unsaturated cycloalkyl radical with 3 to 7 members (such as radicals from cyclopropyl to cycloheptyl, cyclohex-1-enyl) and the ring may comprise one or more oxygen, sulfur or nitrogen atoms (such as 2-tetrahydrofuranyl, 1-piperidinyl, 1-pyrrolidinyl) and each ring member may be mono or disubstituted with substituents such as halo, hydroxy, (C₁-C₄)alkyloxy, thio and (C₁-C₄)-alkylthio), (C₁-C₄)-alkanesulfonyl, amino, (C₁-C₄)-alkylamino and di-(C₁-C₄)-alkylamino, (C₁-C₄)-alkyloxycarbonyl, cyano, oxo,
   e) an aromatic or heteroaromatic five- or six-membered ring (such as phenyl, furyl, 2-pyridyl),
   f) a hydroxy, (C₁-C₁₀)-alkyloxy (such as methoxy, ethoxy, n-propoxy, isopropoxy, tert-butoxy, cyclohexyloxy), mono or disubstituted (C₁-C₁₀)-alkyloxy (such as 2-hydroxyethyloxy), 2,3-dihydroxyprop-1-yloxy, 2-methoxyethyloxy, fluoromethyloxy, aminoethyloxy, 2-dimethylaminoethyloxy), acyloxy (such as formyloxy, acetyloxy, benzoyloxy, ethoxycarbonyloxy, allyloxycarbonyloxy), mono, di or tri-(C₁-C₄)-alkylsilyloxy (such as trimethylsilyloxy, tert-butyldimethylsilyloxy) group,
   g) a mercapto, (C₁-C₁₀)-alkylmercapto (such as methylinercapto, ethylmercapto), mono or disubstituted (C₁-C₁₀)-alkylmercapto (such as 2-hydroxyethylmercapto, 2,3-dihydroxypropl-ylmercapto, 2-methoxyethylinercapto, 2-mercaptoethylmercapto, 2-methylmercaptoethylmercapto, 2-aminoethylmercapto, 2-dimethylaminoethylmercapto), acylmercapto (such as acetylimercapto) group,
   h) an amino, (C₁-C₄)-alkylamino and di-(C₁-C₄)-alkylamino (such as methylamino, dimethylamino, 2-hydroxyethylamino, bis(2-hydroxyethyl)amino, aminoethyloxy, 2-dimethylaminoethyloxy, 2-aminoethylamino, 2-piperidinoethylamino), acetylamino, allyloxycarbonylamino, iminomethylamino, N-methylaminomethylene amino, N,N-dimethylaminomethyleneamino, guanidino, cyanoguanidino, methylguanidino group,
   i) a halo atom (such as fluoro, chloro, bromo, iodo),
   j) an azido, nitro, cyano, (C₁-C₄)alkyloxycarbonyl (such as carbomethoxy, carbethoxy) group,
   k) a (C₁-C₄)-alkanesulfonyl group (such as methanesulfonyl, ethanesulfonyl);
ii) one or more carbon atoms in the ring marked C may be substituted with a substituted or unsubstituted alkyl chain which is linked to the ring marked C via double bond in the form of >C*=CR¹R², and C* means a carbon atom in the ring marked, C= means an exocyclic double bond and the substituents R¹ and R², which may be the same or different, may mean:
   a) a hydrogen atom so that the substituent is methylene,
   b) an unsubstituted saturated alkyl chain with 1 to 20 carbon atoms and the unsubstituted saturated alkyl chain may be straight (such as ethylidene, n-propylidene, n-butylidene, isopropylidene) or branched in any position (such as 2,2-dimethylpropylidene),
   c) an unsubstituted unsaturated alkyl chain with 1 to 20 carbon atoms and the unsubstituted unsaturated alkyl chain may be straight or branched with double bonds or triple bonds (such as vinylidene, allylidene),
   d) an unsubstituted saturated or partly unsaturated cycloalkyl or heteroaryl with 3 to 7 members (as comprised in groups from cyclopropylmethylene to cycloheptylmethylene),
   e) an aromatic or heteroaromatic five- or six-membered ring (as comprised in groups such as benzylidene, diphenylmethylene),
   f) a substituted saturated or partly unsaturated alkyl chain or substituted three- to seven-membered carbocyclic ring whereat any carbon atom in the chain or in the ring may be mono or disubstituted with substituents such as halo, hydroxy, (C₁-C₄)-alkyloxy, (C₁-C₄)-alkylthio, (C₁-C₄)-alkanesulfonyl, amino, (C₁-C₄)-alkylamino, di-(C₁-C₄)-alkylamino, (C₁-C₄)-alkyloxycarbonyl, cyano, oxo as comprised in groups such as hydroxyethylidene, methoxyethylidene,
   g) a hydroxy, (C₁-C₄)-alkyloxy, acyloxy, mercapto, (C₁-C₄)-alkylmercapto, amino, (C₁-C₄)-alkylamino, di-(C₁-C₄)-alkylamino and acylamino group as comprised in groups such as hydroxymethylene, methoxymethylene, dimethoxymethylene, dimethylaminomethylene, acetylaminomethylene groups,
   h) a nitro, cyano, (C₁-C₄)-alkyloxycarbonyl group so that the substituent is dicyanomethylene, bis (carbomethoxy)methylene, carbethoxymethylene;
      and the substituents R¹ and R² may also mean a joint alkylene chain (CH₂)ₙ (n = 2 to 7) closed to a ring and any methylene (-CH₂-) member may be replaced by oxa (-O-), thia (-S-), imino (-NH-) or (C₁-C₄)-alkylimino group as comprised in groups such as cyclopentylidene, cyclohexylidene, 2-(1,3-dioxacyclopentylidene);
iii) one or more carbon atoms in the ring marked C may be substituted with a hetero atom via double bond (such as oxo, thioxo, hydroxyimino, (C₁-C₄)-alkylimino, acylimino);
iv) one or more carbon atoms in the ring marked C may be disubstituted with substituents closed to a ring to obtain a spiro compound and, besides a carbon atom, the ring members may also be oxygen, sulfur and nitrogen atoms (such as ethylene, 1,3-propylene, 1,5-pentylene, ethylenedioxy);
v) one or more carbon atoms in the ring marked C may be replaced by an oxygen atom;
vi) one or more carbon atoms in the ring marked C may be replaced with a sulfur atom which may be mono or dioxidized to obtain sulfoxides or sulfones;
vii) one or more carbon atoms in the ring marked C may be replaced with a nitrogen atom which may be substituted in the form of >N*-R³ and N* means a nitrogen atom in the ring marked C and R³ may mean:
   a) a saturated alkyl chain with 1 to 20 carbon atoms and the unsubstituted saturated chain may be straight (such as methyl, ethyl, n-propyl, n-butyl) or branched in any position (such as isopropyl, s-butyl, isobutyl, isoamyl, tert-butyl) and each chain member may be once or several times substituted with substituents such as halo (such as fluoromethyl, trifluoromethyl, 2-chloroethyl), hydroxy (such as 2-hydroxyethyl), (C₁-C₄)-alkyloxy (such as 2-methoxyethyl), (C₁-C₄)-alkylthio (such as methylmercaptoethyl), (C₁-C₄)-alkanesulfonyl (such as methanesulfonylmethyl), amino, (C₁-C₄)-alkylamino, di-(C₁-C₄)-alkylamino (such as 2-aminoethyl, 2-methylamino ethyl, 2- dimethylaminoethyl), aromatic or heteroaromatic five-or six-membered ring (such as phenyl, furyl, 2-pyridyl), (C₁-C₄)-alkyloxycarbonyl (such as carbethoxymethyl), cyano (such as cyanoethyl), oxo (such as acetyl, propionyl, 2-oxopropyl), imino (such as iminomethyl, aminoiminomethyl, aminocyanoiminomethyl),
   b) an unsubstituted unsaturated alkyl chain with 1 to 20 carbon atoms and this unsubstituted unsaturated alkyl chain may be straight with double bonds (such as vinyl, propenyl, allyl) or triple bonds (such as ethinyl, propargyl) or branched in any position with double or triple bonds (such as 2-propenyl),
   c) an unsubstituted saturated or partly unsaturated cycloalkyl or heteroaryl radical with 3 to 7 members (such as radicals from cyclopropyl to cycloheptyl, cyclohex-1-enyl, 4-piperidinyl),
   d) an unsubstituted aromatic or heteroaromatic five- or six-membered ring (such as phenyl, furyl, 2-pyridyl),
   e) groups such as cyano, (C₁-C₄)-alkyloxycarbonyl (such as carbomethoxy, carboethoxy), aminocarbonyl, (C₁-C₄)-alkylaminocarbonyl, (C₁-C₄)-alkylsulfonyl (methanesulfonyl);
      and wherein X may mean:
      i. a hydrogen atom so that the compound of the Formula (I) is a carboxylic acid; in C1 the case of a basic centre in the molecule, a hydrogen atom is linked to it as a proton, the carboxyl group is in the anion form as carboxylate and the compound of the Formula (I) is in the form of a zwitter ion,
      ii. an alkali metal so that the compound of the Formula (I) is an alkali metal salt (such as lithium carboxylate, sodium carboxylate, potassium carboxylate),
      iii. an earth alkali metal so that the compound of the Formula (I) is an earth alkali metal salt wherein for one bivalent metal ion there are two carboxylate anions (such as calcium dicarboxylate),
      iv. the ammonium ion or a protonated form of mono, di or trisubstituted acyclic or cyclic aliphatic amine or a protonated form of some other nitrogen base,
         the compound of Formula (I) is in this case a salt of a carboxylic acid and ammonia or amine (such as trimethylamine, triethylamine, N,N'-dibenzylethylene diamine, ethanolamine, diethanolamine, triethanolamine, dicyclohexylamine, 2-piperidinyl) or amidine (such as 1,8-diazabicyclo[5.4.0]undec7-ene, 1,5-diazabicyclo[4.3.0]non-5-ene, 3,3,6,9,9-pentadimethyl-2,10-diazabicyclo[4.4.0]dec-1-ene) or guanidine (such as guanidine, cyanoguanidine) or some other nitrogen base (such as 4-dimethylaminopyridine, imidazole).
      v. the quatemized ammonium ion so that the compound of the Formula (I) is a corresponding quaternary ammonium carboxylate (such as tetrabutylammonium carboxylate),
      vi. a radical R⁴ whereat the compound of the Formula (I) is in the ester form and the radical R⁴ may be:
         a) selected from the group comprising (C₁-C₂₀)-alkyl (such as methylethyl, tert-butyl), (C₁-C₂₀)-alkenyl (such as allyl), substituted alkyl (such as (C₁-C₄)-alkoxyalkyl, (C₁-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc.,
         b) a radical which may be presented in a following form 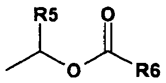 wherein
            R⁵ represents hydrogen or a lower alkyl with 1 to 4 carbon atoms,
            R⁶ represents hydrogen, alkyl cycloalkyl, alkoxy, cycloalkoxy, cycloalkylalkyl, alkenyloxy, phenyl.

Further on, described are ethylene derivatives of tricyclic carbapenems of the general Formula (I) in the form of pure diastereoisomers and in the form of pure geometric isomers. The compounds of the Formula (I) comprise at least 2 pure geometric isomers when the methyl group in the ethylidene substituent in 6 position of the carbapenem ring of the compound of the Formula (I) is configured around a double bond as (Z) or as (E) and at least 2 pure diastereoisomers since a new chiral centre in 4 position, which is formed in a joint point with the new ring, may be configured as (R) or as (S).

In Scheme 1 and in all further schemes the bold bond represents the position above the level of the sheet and the broken line represents the position under the level of the sheet. The mark (R) or (S) depends on the kind of ring marked C and on the substituents bound to the ring marked C and is determined according to Cahn-Ingold-Prelog rule (Calin et al., Experientia 12, (1956) 81).

The configuration in 5 position in the joint point of the four- and five- ring of the compound of the general Formula (I) is always the same and is always under the level of the sheet and the mark (R) or (S) is determined according to the above-mentioned Cahn-Ingold-Prelog rule.

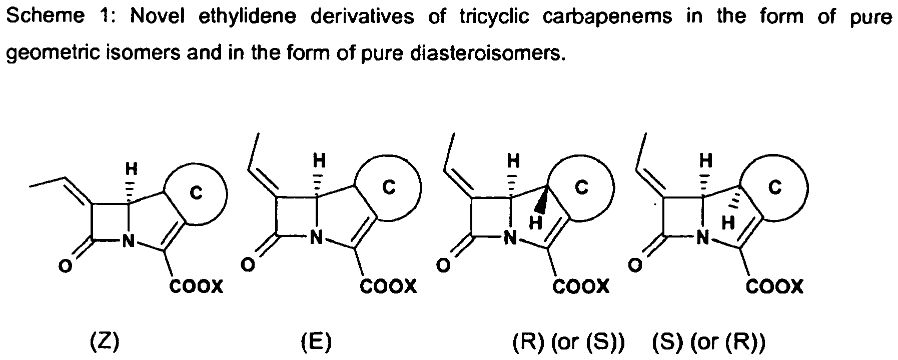

The present invention is also directed to the use of a therapeutical effective amount of ethyliden derivatives of tricyclic carbapenem of Formula (I) as defined in the claims or a salt thereof as a broad spectrum β-lactamase inhibitor.

The following compounds of the Formula (I) are especially important as inhibitors of the action of enzymes β-lactamases and/or as antibiotics:
(8*S*,9*R*)-10-((E)-ethylidene)-11-oxo-I-azatricyclo[7.2.0.0^{3.8}]undec-2-ene-2-carboxylic acid, a pharmaceutically acceptable salt or ester thereof,
(8*R*,9*R*)-10-((E)-ethylidene)11-oxo-I-azatricyclo[7.2.0.0^{3.8}]undec-2-ene-2-carboxylic acid, a pharmaceutically acceptable salt or ester thereof,
(85*S*,9*R*)-10-((E)-ethylidene)-11-oxo-I-aza-6-thiatricyclo[7.2.0.0^{3.8}]undec-2-ene-2-carboxylic acid, a pharmaceutically acceptable salt or ester thereof,
(8*R*,9*R*)-10-((E)-ethylidene)-11-oxo-I-aza-6-thiatricyclo[7.2.0.0^{3.8}]undec-2-ene-2-carboxylic acid, a pharmaceutically acceptable salt or ester thereof.
(4*S*,8*S*,9*R*)-4-methoxy-10-((E)-ethylidene)-11-oxo-1-azatricyclo[7.2.0.0^{3.8}]undec-2-ene-2-carboxylic acid, a pharmaceutically acceptable salt or ester thereof, (thereafter referred to as "**LK-157**"),
(4*R*,8*R*,9*R*)-4-methoxy-10-((E)-ethylidene)-11-oxo-1-azatricyclo[7.2.0.0^{3,8}]undec-2-ene-2-carboxylic acid, a pharmaceutically acceptable salt or ester thereof,
(4*S*,8*S*,9*R*)-4-methoxymethyl-10-((E)-ethylidene)-11-oxo-1-azatricyclo[7.2.0.0^{3.8}]undec-2-ene-2-carboxylic acid, a pharmaceutically acceptable salt or ester thereof,
(4*R*,8*R*,9*R*)-4-methoxymethyl-10-((E)-ethylidene)-11-oxo-1-azatricyclo[7.2.0.0^{3,8}]undec-2-ene-2-carboxylic acid, a pharmaceutically acceptable salt or ester thereof.

It also has been found that compounds of Formula (I), when used in combination with an antibiotic, preferably in combination with β-lactam antibiotics will result in an increased antibacterial activity (synergistic effect) against Class A, Class C and Class D producing organisms.

Thus, the invention also relates to pharmaceutical compositions comprising at least a compound defined in the claims or a pharmaceutically acceptable salt thereof and an antibiotics selected from ceftazidime, cefotaxime, cefepime and cefpirome.

Especially cefepime, cefpirome, ceftazidime or cefotaxime show a broad spectrum of activity against Gram-positive and Gram-negative pathogens.

Thus, the present invention also relates to a pharmaceutical composition as defined above wherein the β-lactam antibiotic cefalosporine is selected from a group consisting of ceftazidime, cefotaxime, cefepime or cefpirome, combinations with a β-lactam antibiotic selected from a group consisting of ceftazidime, cefotaxime, or cefepime being especially preferred.

Also contemplated is the pharmaceutical composition as described above wherein the β-lactam antibiotic penicilline is piperacillin.

Also contemplated is a pharmaceutical composition as mentioned above wherein the β-lactam antibiotic monobactam is aztreonam.

The β-lactam antibiotic carbapenem may be meropenem.

The compounds of the invention are formulated in pharmaceutical compositions by combining the compounds with any conventional non-toxic pharmaceutically acceptable carrier, adjuvants or vehicles.

Thus, the present invention is also directed to a pharmaceutical composition comprising a tricyclic carbapenem of Formula (I) or a salt thereof, a β-lactam antibiotic and a pharmaceutically acceptable carrier, as defined in the claims.

As used herein, the term "pharmaceutically acceptable" means a non-toxic material that does not interfere with the effectiveness of the biological activity of the active ingredient(s). The term "physiologically acceptable" refers to a non-toxic material that is compatible with a biological system such as a cell, cell culture, tissue, or organism.

The term "pharmaceutically acceptable carrier" refers to a non-toxic carrier that may be administered to a patient, together with a compound of this invention in combination with antibiotics, preferably β-lactam antibiotics, and which does not destroy the pharmacological activity thereof.

In general, all carriers known by a respective person skilled in the art are suitable for their use within the present pharmaceutical composition. Normally, the characteristics of the preferred carrier depend on the route of administration of the respective pharmaceutical composition.

Solid carriers which are usable according to the present invention are for example finely divided solids such as talc, clay, microcrystalline cellulose, silica, alumina and the like. Useful liquid carriers include water, alcohols or glycols or water- alcohol/glycol blends, in which the present compounds can be dissolved or dispersed at effective levels, optionally with the aid of non-toxic surfactants. Adjuvants such as fragrances and additional antimicrobial agents can also be added to optimize the properties for a respective use. The resultant liquid pharmaceutical compositions can be applied from an absorbent pad, used to impregnate bandages and other dressings, or sprayed onto the affected area using pump-type or aerosol sprayers.

For topical administration, it will generally be desirable to administer the present compounds to the skin as compositions or formulations, in combination with a dermatologically acceptable carrier, which may be a solid or a liquid. Topical applications may be formulated in carriers such as hydrophobic or hydrophilic bases to form ointments, creams, lotions, in aqueous, oleaginous or alcoholic liquids to form paints or in dry diluents to form powders. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user. Cream or ointment formulations which may be used for the drug are conventional formulations well known in the art.

Further on the present invention refers to a pharmaceutical composition comprising a tricyclic carbapenem of Formula (I) as defined above or a salt, ester or amide thereof, a β-lactam antibiotic and optionally a pharmaceutically acceptable excipient, as defined in the claims.

In general all excipients known by a person skilled in the art are suitable within the present invention. Examples of such excipients are calcium carbonate, kaolin, sodium hydrogen carbonate, lactose, D-mannitol, starches, crystalline cellulose, talc, granulated sugar, porous substances, etc.

The compound of Formula (I) or the salts thereof may be used as bulk itself but usually be formulated into pharmaceutical preparations together with a suitable amount of "carrier for pharmaceutical preparation" according to ordinary methods.

Further on, "carriers for pharmaceutical preparation" comprises, for example, excipients as defined above, binders (e.g., dextrin, gums, α-starch, gelatin, hydroxypropyl cellulose, hydroxypropylmethyl cellulose, pullulan, etc.), thickening agents (e.g., natural gums, cellulose derivatives, acrylic acid derivatives, etc.), disintegrators (e.g., carboxymethyl cellulose, croscarmellose sodium, crospovidone, low-substitution hydroxypropyl cellulose, partial α-starch, etc.), solvents (e.g., water for injections, alcohol, propylene glycol, macrogol, sesame oil, corn oil, etc.), dispersants (e.g., Tween 80, HCO60, polyethylene glycol, carboxymethyl cellulose, sodium alginate, etc.), solubilizers (e.g., polyethylene glycol, propylene glycol, D-mannitol, benzyl benzoate, ethanol, trisaminomethane, triethanolamine, sodium carbonate, sodium citrate, etc.), suspending agents (e.g., stearyl triethanolamine, sodium lauryl sulfate, benzalkonium chloride, polyvinyl alcohol, polyvinylpyrrolidone, hydroxyethyl cellulose, etc.), pain-reducing agents (e.g., benzyl alcohol, etc.), isotonizing agents (e.g., sodium chloride, glycerin, etc.), buffers (e.g., phosphates, acetates, carbonates, citrates, etc.), lubricants (e.g., magnesium stearate, calcium stearate, talc, starch, sodium benzoate, etc.), colorants (e.g., tar pigments, caramel, iron sesquioxide, titanium oxide, riboflavins, etc.), tasting agent (e.g., sweeteners, flavors, etc.), stabilizers (e.g., sodium sulfite, ascorbic acid, etc.), preservatives (e.g., parabens, sorbic acid, etc.), and the like.

Compositions and methods according to the invention may also contain additionally diluents, fillers, salts, buffers, stabilizers, solubilizers, and other materials well known in the art. Pharmaceutical compositions according to the present invention may also comprise other active factors and/or agents which enhance the inhibition of β-lactamases and/or DD-peptidases.

Respective pharmaceutical compositions according to the present invention are effective against bacteria which do not produce β-lactamases, but also especially effective against bacteria which produce significant amounts of β-lactamases. Thus, pharmaceutical compositions according to the present invention are generally useful for controlling bacterial infections levels *in vivo* and for treating diseases or reducing the advancement or severity of effects, which are mediated by bacteria.

Suitable subjects for the administration of the formulation of the present invention include mammals, primates, man, and other animals. Typically the animal subject is a mammal, generally a domesticated farm mammal (e.g. horse, pig, cow, sheep, goat etc.) or a companion animal (e.g. cat, dog etc.). *In vitro* antibacterial activity is predictive of *in vivo* activity when the compositions are administered to a mammal infected with a susceptible bacterial organism.

### Route of administration

Preferred methods of administration of the pharmaceutical compositions described above include oral and parenteral, e.g., i.v. infusion, i.v. bolus and i.m. injection formulated so that a unit dosage comprises a therapeutically effective amount of each active component or some submultiples thereof.

The compounds may be employed in powder or crystalline form, in liquid solution, or in suspension. Theses compounds may be formulated by any method well known in the art and may be prepared for administration by any route, including, without limitation, parenteral, oral, sublingual, by inhalation spray, transdermal, topical, intranasal, intratracheal, intrarectal via ophthalmic solution or ointment, rectally, nasally, buccally, vaginally or via implanted reservoir. The term parenteral as used herein includes subcutaneous, intracutaneous, intravenous, intramuscular, intra-articular, intrasynovial, intrasternal, intrathecal, intralesional and intracranial injection or infusion techniques.

Pharmaceutical compositions for injection, a preferred route of delivery according to the present invention, may be prepared in unit dosage form in ampules, or in multidose containers. The composition will generally be sterile and pyrogen-free, when intended for delivery by injection into the subject. The injectable compositions may take such forms as suspensions, solutions, or emulsions in oily or aqueous vehicles, and may contain various formulating agents. Alternatively, the active ingredient may be in powder (lyophilized or non-lyophilized) form for reconstitution at the time of delivery with a suitable vehicle, such as sterile water.

Carriers suitable for injectable pharmaceutical composition according to the present invention are typically comprised sterile water, saline or another injectable liquid, e.g., peanut oil for intramuscular injections. Also, various buffering agents, preservatives and the like can be included. The active ingredient(s) may also be administered parenterally in a sterile medium. Depending on the vehicle and concentration used, the drug can either be suspended or dissolved in the vehicle. Advantageously, adjuvants such as local anaesthetic, preservative and buffering agents can be dissolved in the vehicle. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. It is also preferred to include isotonic agents, for example, sugars, buffers or sodium chloride. Prolonged absorption of the injectable compositions can be brought about by the use in the compositions of agents delaying absorption, for example, aluminium monostearate and gelatine. Intra-venous infusion is another possible route of administration for the compounds used according to the present invention.

Orally administrable pharmaceutical compositions according to the present invention may be in the form of tablets, capsules, powders, granules, lozenges, liquid or gel preparations, such as oral, topical, or sterile parenteral solutions or suspensions. The oral compositions may utilize carriers such as conventional formulating agents, and may include sustained release properties as well as rapid delivery forms. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

Tablets and capsules for oral administration may be in unit dose presentation form, and may also contain conventional excipients such as binding agents, for example syrup, acacia, gelatine, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrates for example potato starch, or acceptable wetting agents such as sodium lauryl sulphate. The tablets may be coated according to methods well known to a person skilled in the art. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatine hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

Pharmaceutical compositions according to the present invention may also be prepared in suitable forms for absorption through the mucous membranes of the nose and throat or bronchial tissues and may conveniently take the form of powder or liquid sprays or inhalants, lozenges, throat paints, etc. For medication of the eyes or ears, the preparations may be presented as individual capsules, in liquid or semi-solid form, or may be used as drops, etc.

For veterinary medicine, the composition may, for example, be formulated as an intramammary preparation in either long acting or quick-release bases.

### Use of the inhibitor as broad spectrum β-lactamase inhibitor

There are also described novel β-lactamase inhibitors of Formula (I) described above which are structurally unrelated to the natural product and semi-synthetic β-lactamase inhibitors presently available on the market. Certain embodiments of these new inhibitors also bind bacterial DD-peptidases, and thus may potentially act both as β-lactamase inhibitors and as antibiotic agents.

Compounds of Formula (I) are especially suitable as β-lactamase inhibitors for therapeutic applications. They are also useful as pharmacological tools for *in vitro* or *in vivo* studies to investigate the mechanisms of antibiotic resistance, to help identify other therapeutic antibiotic agents or β-lactamase inhibitors, to identify which β-lactamases are being expressed by a given microorganism, or to selectively inhibit one or more β-lactamases in a microorganism.

Thus, also contemplated is the use of a therapeutically effective amount of ethylidene derivatives of tricyclic carbapenem of Formula (I) as defined above or a salt, ester or amide derivate thereof as a broad spectrum β-lactamase inhibitor.

The β-lactamase inhibitor may be a broad spectrum inhibitor of Class A, C and D βlactamases. It effectively inhibits most of the clinically relevant and prevalent TEM- and SHV-type enzymes (Class A), AmpC (Class C) and OXA-type enzymes (Class D).

Broad spectrum of β-lactamase inhibitor and careful selection of β-lactam antibiotic in combination is superior to current therapeutic options.

### Inhibition of Bacterial Growth

In a further aspect, methods for inhibiting bacterial growth are contemplated, such methods comprising administering a β-lactamase inhibitor of Formula (I) in combination with antibiotics, preferably a β-lactam antibiotics as defined above to a bacterial cell culture, or to a bacterially infected cell culture, tissue, or organism.

It is known that the response to a given combination may be strain specific and is not solely related to the level of sensitivity/resistance to the specific members of the combination. Thus, the combinations of the present invention are intended to be useful on all bacterial strains including those not mentioned herein.

Preferably, the bacteria to be inhibited by administration of a β-lactamase inhibitor described herein are bacteria that are resistant to β-lactam antibiotics. More preferably, the bacteria to be inhibited are β-lactamase positive strains that are highly resistant to β-lactam antibiotics. The terms "resistant" and "highly resistant" are well-known by those of ordinary skill in the art.

Polymicrobial infections often include pathogens that produce β-lactamase enzymes. These enzymes commonly cause resistance to penicillins and cephalosporins. Without treatment these microbes would multiply and thrive unimpeded, with serious or critical consequences to the patient.

Thus, also contemplated are pharmaceutical compositions and methods for overcoming bacterial antibiotic resistance.

### Method of treatment

The compositions are useful for inhibiting bacterial growth in a variety of contexts. In a preferred embodiment, the compound of Formula (I) as defined above is administered to an experimental cell culture *in vitro* to prevent the growth of β-lactam resistant bacteria. According to another preferred embodiments the compound of Formula (I) as defined above is administered to an animal, including a human, to prevent the growth of β-lactam resistant bacteria *in vivo.* The method according to this embodiment comprises administering a therapeutically effective amount of a β-lactamase inhibitor described herein for a therapeutically effective period of time to an animal, including a human.

Thus, also contempled is a method of inhibiting β-lactamase comprising contacting the β-lactamase with an effective amount of the compound of ethylidene derivatives of tricyclic carbapenem of Formula (I) defined above or a salt, ester or amide derivative thereof.

Also contemplated is a a method of treatment of a bacterial infection in a human or animal subject wherein the method comprising administering to the subject in need thereof a therapeutically effective amount of ethylidene derivatives of tricyclic carbapenem of Formula (I) defined above or a salt, ester or amide derivative thereof, and an antibiotic, preferably a β-lactam antibiotic.

Thus, the present invention is also directed towards the use of a pharmaceutical composition as defined in the claims for the treatment of an infection in humans or animals caused by a bacterium.

Safe and effective dosages for different Classes of patients and for different disease states will be determined by clinical trial as is required in the art. The specific dosage and treatment regimens for any particular patient will depend upon a variety of factors, including the activity of the specific compound employed, the age, body weight, general health status, sex, diet, time of administration, route and frequency of administration, rate of excretion, drug combination, the sensitivity of the pathogen to the particular compound selected, the virulence of the infection, the severity and course of the disease, and the patient's disposition to the disease. Such matters, however, are left to the routine discretion of the physician according to principles of treatment well known in the antibacterial arts.

The terms "therapeutically effective amount" and "therapeutically effective period of time" are used to denote known treatments at dosages and for periods of time effective to show a meaningful patient benefit, i.e., healing of conditions associated with bacterial infection, and/or bacterial drug resistance. Preferably, such administration should be parenteral, oral, sublingual, transdermal, topical, intranasal, intratracheal, or intrarectal. When administered systemically, the therapeutic composition is preferably administered at a sufficient dosage to attain a blood level of inhibitor of at least about 100 micrograms/mL, more preferably about 1 milligrams/mL, and still more preferably about 10 milligrams/mL. For localized administration, much lower concentrations than this may be effective, and much higher concentrations may be tolerated.

In case of co-administration of a compound of Formula (I) as defined above or a salt, ester or amide derivative thereof with an antibiotic, most preferably a β-lactam antibiotic, the ratio of the amount of the compound to the amount of the antibiotic, most preferably a β-lactam antibiotic may vary in a wide range.

The ratio of β-lactam antibiotic to β-lactamase inhibitor may vary from 1:1 to 100:1. Preferably the ratio of the β-lactam antibiotic to β-lactamase inhibitor is less than 10:1.

The novel pharmaceutical compositions according to the present invention for human delivery per unit dosage, whether liquid or solid, comprise from about 0.01% to as high as about 99% of the ethylidene derivatives of tricyclic carbapenems of Formula (I) as defined in the claims or derivative thereof, such as a salt, ester or amide. The preferred range being from about 10-60% and from about 1% to about 99.99% of one or more of other antibiotics such as those discussed herein, preferably from about 40% to about 90%.

The pharmaceutical composition will generally contain from about 50 mg to about 2.0 g of the ethylidene derivatives of tricyclic carbapenems of Formula (I) as defined in the claims or salt, thereof However, in general, it is preferable to employ dosage amounts in the range of from about 250 mg to 1000 mg and from about 50 mg to about 5 g of the other antibiotics discussed herein; preferably from about 250 mg to about 2000 mg.

In parenteral administration, the unit dosage will typically include the pure compound of Formula (I) in sterile water solution or in the form of a soluble powder intended for solution, which can be adjusted to neutral pH and isotonic. For children, a dose of about 5-25 mg/kg of body weight given 2, 3, or 4 times per day is preferred; a dose of 10 mg/kg is typically recommended.

According to a special embodiment of the use according to the present invention, a β-lactamase inhibitor according to the invention is co-administered with an antibiotic, preferably with a β-lactam antibiotic.

For purposes of this invention, the term "co-administered" is used to denote simultaneous or sequential administration. Preferably, such co-administration produces a synergistic effect. As employed herein, the terms "synergy" and "synergistic effect" indicate that the effect produced when two or more drugs are co-administered is greater than would be predicted based on the effect produced when the compounds are administered individually.

β-lactamase inhibitors according to the present invention act to prevent degradation of β-lactam antibiotics, thereby enhancing their efficacy and producing a synergistic effect. Thus, according to special embodiment of the present invention the co-administered antibiotic is a β-lactam antibiotic.

In a further special embodiment of the present invention the compounds of the present pharmaceutical composition are co-administered with cephalosporin, such as cefepime, cefpirome, ceftazidime or cefotaxime intravenously.

The compounds of the present pharmaceutical composition may be provided prior to, simultaneously with, or subsequent to a β-lactam antibiotic ("co-administration"). The two active components may be administered separately by different routes, if desired.

In a further special embodiment of the present invention the two active agents will be administered by the same route and preferably in a single composition, so as to ensure that they are given simultaneously to the subject.

The invention is further described in connection with the following examples.

### EXAMPLE 1

### Materials and Methods

### IC₅₀ Determination for the Inhibitors

The IC₅₀ value represents the concentration of inhibitor required to effect a 50% loss of activity of free enzyme. A standard test for the production of β-lactamase involves use of the chromogenic cephalosporin, nitrocefin. This compound exhibits a rapid distinctive colour change from yellow (maximum OD at pH 7.0 at lambda 390 nm) to red (maximum OD at pH 7.0, at lambda 486 nm), as the amide bond in the β-lactam ring is hydrolysed by a β-lactamase.

Homogeneously purified Class A β-lactamases TEM-1 and SHV-1 from *E*. *coli* and Class C enzyme AmpC from *Enterobacter Cloaca* were employed in the assay.

Compounds were prepared as 50 mM stocks in DMSO and diluted with buffer P1 (50 mM phosphate, pH 7) to a final concentration of 10% DMSO. All further dilutions were done using P2 (P1 with 10% DMSO). Enzyme and compound dilutions were pre-incubated for 10 min at 37°C and the reaction started with the addition of pre-warmed (37°C) nitrocefin (reporter substrate) at a final concentration of 50 mM. The change in absorption at 490 nm was followed at 37°C for 10 min at 30 sec intervals in a SPECTRAMAX 384 PLUS microplate reader (Molecular Devices, Sunnyvale, CA) using 96-well plates. The initial rate was determined and IC₅₀ values were calculated using non-linear regression and sigmoidal dose response analysis with the PRISM 4.0 software (Graphpad Software Inc., San Diego, CA). IC₅₀ data is expressed as µM and was calculated from at least two independent experiments.

Representative compounds of Formula (I) were also evaluated as β-lactamase inhibitors of TEM-1 and SHV-1 (Class A, penicillinase) from *E*. *coli* and P-99 (Class C, cephalosporinase) from *Enterobacter Cloacae,* by relative IC₅₀ analysis using a procedure similar to that described above. The data is presented in Table 1 below.

**Table I: In vitro IC₅₀s activity and calculated partition coefficients of tricyclic carbapenems of Formula (I) 1a-1h against Class A (TEM-1 and SHV-1) and Class C (AmpC) β-tactamases.**

| | | **IC₅₀ [µM]** | | |
|---|---|---|---|---|
| **Compound** | **R** | **TEM-1** | **SHV-1** | **AmpC** |
| **clavulanate** | | 0.030 | 0.028 | 136.2 |
| **tazobactam** | | 0.017 | 0.222 | 1.808 |
| **1a** (4*S*,8*S*) | - Me | 0.055 | 0.151 | 0.062 |
| **1b** (4*R*,8*R*) | - Et | 0.022 | 0.284 | 0.295 |
| **1c** (4*S*,8*S*) | - Et | 0.057 | 0.385 | 0.229 |
| **1d** (4*R*,8*R*) | - iPr | 0.039 | 0.340 | 0.341 |
| **1e** (4*R*,8*R*) | - Bu | 0.012 | 0.219 | 0.430 |
| **1f** (4*S*,8*S*) | - Bu | 0.032 | 0.206 | 0.720 |
| **1g** (4*S*,8*S*) | - (CH₂)₂F | 0.021 | 0.148 | 1.020 |
| **1h** (4*S*,8*S*) | - (CH₂)₂CN | 0.018 | 0.152 | 0.209 |

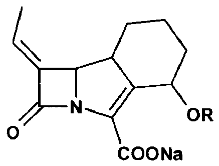

LK-157 is a new chemical entity (4*S*,8*S*,9*R*)-4-methoxy-10-((E)-ethylidene)-11-oxo-1-azatricyclo[7.2.0.0^{3.8}]undec-2-ene-2-carboxylic acid (compound **1a**), a pharmaceutically acceptable salt or ester thereof, (thereafter referred to as "**LK-157**").

It was selected from a series of new ethylidene derivatives of tricyclic carbapenems as a promising candidate for a β-lactamase inhibitor to be co-administered with a selected β-lactam antibiotic.

### EXAMPLE 2

### MIC Determinations in the Presence and Absence of β-Lactamase Inhibitor

### Antibiotics

Combinations of 3 different β-lactam antibiotics (ceftriaxone, ceftazidime and, cefotaxime) and β-lactamase inhibitor of Formula (I) with constant concentration ratio (2:1) were tested and compared to cephalosporin alone and Tazocin^{®}.

### Bacterial strains

All tested strains and Clinical isolates were either purchased from the American Type Culture Collection (ATCC) or from the in-house company culture collection. The tested strains were purely used for the purposes of illustrative example, they are by no means essential for performing the invention.

### Cultivation and Maintenance of Test Organisms

The strains were processed according to procedures recommended by ATCC, or procedures that are routinely used. Frozen bacterial stocks were thawed to room temperature, and a few drops were placed on an appropriate plate; chocolate agar was used for *H. influenzae,* blood agar was used for all others. The cultures were subcultured on a fresh Mueller-Hinton agar plate (MHA) the following day, and the subcultures were again incubated overnight.

### Inoculum

Bacterial suspensions with a turbidity equivalent to that of a 0.5 McFarland standard were prepared by suspending a tiny portion of one colony from blood agar plates in 2 mL of sterile saline. Suspensions were further diluted with cation adjusted Mueller Hinton Broth (CAMHB) to obtain a final inoculum of 5x 10⁵ CFU/ mL.

### Broth Microdilution Procedure

The MIC experiments were performed in duplicate in 96-well microtiter plates using CAMHB. Serial twofold dilutions of each antibiotic either alone or in combination with constant concentration ratio of a β-lactamase inhibitor (2:1) were prepared in CAMHB. The bacterial suspension with final inoculum 10⁵ CFU/mL was transferred to the test medium containing the antibacterial substances. In each well of a 96-well microtiter plate was combined 50 µL of bacterial inoculum, 50 µL of antibiotic dilutions (or media for determination of the MIC of the antibiotic in the absence of test compound). Each plate included 4 wells with no bacterial inoculum (negative control) and 4 wells with no test compound and no antibiotic (positive control). The plates were incubated at 35°C for 24 h. Purity check and colony counts on each inoculum suspension was performed to ensure that the final inoculum concentration routinely obtained closely approximates 5 x 10⁵ CFU/ mL. In addition the assay is routinely monitored by testing standard antibiotics and ensuring that MIC values are within the recommended ranges for the respective strains.

The minimal inhibitory concentration (MIC) for all isolates was defined as the lowest concentration of antimicrobial agent that completely inhibits the growth of the organism as detected by the unaided eye. This test permits comparisons to be made between bacterial growth in the presence of antibiotic alone and bacterial growth in the presence of both an antibiotic and a novel (test) compound. Representative results are presented in Table 2.

**Table 2. MIC values of ceftazidime, ceftriaxone, cefotaxime and respective combinations ceftazidime/LK-157, ceftriaxone/LK-157 and cefotaxime/LK-157.**

| | piperacilin + tazobactam (8 1) | ceftazidime (µg/ml) | ceftazidme + LK-157 (2 1) | ceftnaxone (ug/ml) | ceftnaxone LK-157 (2 1) | cefotaxime (µg/ml) | cefotaxime + LK-157 (2 1) |
|---|---|---|---|---|---|---|---|
| Enterobacter cloacae ATCC 2335 | 2 | 4 | 1 | 2 | 2 | 4 | 0.5 |
| | 2 | 4 | 2 | 2 | 05 | 2 | 1 |
| Enterobacter aerogenes ATCC 29751 | 8 | 4 | 2 | 4 | 1 | 2 | 1 |
| | 8 | 2 | 1 | 2 | 05 | 2 | 1 |
| Enterobacterdoacae Lek 10 | 1 | 0.125 | 0.125 | 0.031 | 0.016 | 0.0625 | 0.0625 |
| | 1 | 0.125 | 0.125 | 0.016 | 0.016 | 0.031 | 0.031 |
| Enterobacter cloacae Lek-12 | 128 | 128 | 32 | >128 | 32 | >128 | 64 |
| | 128 | 128 | 32 | >128 | 64 | >128 | 64 |
| Enterobacter cloacae Lek-24 | 128 | >128 | >128 | >128 | 8 | 128 | 4 |
| | 64 | >128 | >128 | >128 | 4 | 128 | 8 |
| Enterobacter cloacae Lek-14 derepressed | 64 | 128 | 4 | >128 | 32 | 128 | 4 |
| | 64 | 128 | 2 | >128 | 32 | 128 | 4 |
| Enterobacter cloacae Lek-33 | 128 | >128 | >128 | >128 | 4 | >128 | 32 |
| | 128 | >128 | >128 | >128 | 4 | >128 | 32 |
| Enterobacter cloacae Lek-30. derepressed | 128 | >128 | >128 | >128 | 8 | >128 | 8 |
| | 128 | >128 | >128 | >128 | 32 | >128 | 32 |
| Enterobacter aerogenes Lek-31. derepressed | 64 | >128 | >128 | 4 | 0.5 | 4 | 1 |
| | 128 | >128 | 128 | 4 | 0.5 | 4 | 1 |
| | | | | | | | |
| Klebsiella oxytoca ATCC 51983 | 8 | 64 | 1 | 8 | 0.25 | 2 | 0.5 |
| | 8 | 32 | 2 | 4 | 0.25 | 8 | 1 |
| Klebsella pneumoniae ATCC 700603 | 16 | 32 | 8 | 2 | 1 | 1 | 0.5 |
| | 16 | 32 | 4 | 2 | 1 | 1 | 1 |
| Klebsiella pneumoniae ATCC 51503 | 32 | >128 | 64 | 4 | 2 | 64 | 0.5 |
| | 16 | >128 | 32 | 2 | 1 | 64 | 0.5 |
| Klebsiella pneumoniae Lek 8 | 32 | 256 | 64 | 8 | 2 | 4 | 4 |
| | 32 | 256 | 16 | 8 | 4 | 4 | 1 |
| Klebsiella pneumoniae Lek-13 | 64 | >128 | 64 | 4 | 0.016 | 0.5 | 0.008 |
| | 32 | >128 | 64 | 4 | 0.031 | 0.5 | 0.002 |
| Klebsiella pneumoniae Lek-18 | 64 | >128 | 4 | 32 | 8 | 64 | 0.5 |
| | 64 | >128 | 4 | 32 | 8 | 64 | 0.5 |
| Klebsiella pneumoniae Lek-32 | >128 | >128 | >128 | 64 | 0.125 | 32 | 0.25 |
| | >128 | >128 | >128 | 32 | 0.125 | 32 | 0.5 |
| Klebsiella pneumoniae Lek-22 | 128 | >128 | 128 | >128 | 64 | >128 | 64 |
| | 128 | >128 | 128 | >128 | >128 | >128 | 64 |
| Klebsiella pneumoniae Lek-29 | 128 | >128 | 128 | >128 | 64 | >128 | 128 |
| | 128 | >128 | >128 | >128 | 64 | >128 | >128 |
| Klebsiella pneumoniae Lek-36 | >128 | >128 | 128 | >128 | >128 | >128 | 64 |
| | >128 | >128 | 64 | >128 | 128 | >128 | 64 |
| | | | | | | | |
| Citrobacter freundₗₗ Lek 3 | 2 | 0.5 | 0.25 | 0.125 | 0.0625 | 0.125 | 0.125 |
| | 1 | 0.25 | 0.125 | 0.0625 | 0.0625 | 0.0625 | 0.0625 |
| Citrobacter freundₗₗ Lek 6 | 1 | 0.125 | 0.125 | 0.0625 | 0.0625 | 0.031 | 0,0625 |
| | 1 | 0.125 | 0.125 | 0.125 | 0.016 | 0.0625 | 0.0625 |
| Citrobacter freundₗₗ Lek9 | 32 | 0,125 | 0.125 | 0.25 | 0.0625 | 16 | 8 |
| | 32 | 0.125 | 0.125 | 1 | 0.031 | 8 | 4 |
| Citrobacter freundₗₗ B318 | 32 | 128 | 2 | 32 | 2 | 4 | 1 |
| | 32 | 128 | 2 | 32 | 2 | 4 | 1 |
| Citrobacterfreundₗₗ Lek-17 | 64 | >128 | 8 | 4 | 0.25 | 4 | 0.25 |
| | 64 | >128 | 8 | 4 | 0.5 | 4 | 0,125 |
| Citrobacter freundₗₗ Lek-34 | 128 | >128 | 32 | 128 | 8 | >128 | 64 |
| | 128 | >128 | 32 | 128 | 4 | >128 | 128 |
| Citrobacter freundₗₗ Lek 4. derepressed | >128 | >128 | 64 | 64 | 32 | 64 | 16 |
| | >128 | >128 | 64 | 64 | 16 | 64 | 16 |

Strains *Enterobacter Cloacae* ATCC 23355 and *Enterobacter aerogenes* ATCC 29751 are known to be sensitive to 3^{rd} generation cephalosporins and produce inducible cephalosporinase. In combinations with LK-157 there was some additional lowering of MICs observed.

1 clinical isolate of *Enterobacter Cloacae* producing AmpC-like enzymes (group 1 β-lactamases) was susceptible to cephalosporins and another 3 were resistant. Also 3 strains producing derepressed AmpC β-lactamase were considered as resistant. Commercial β-lactamase inhibitors generally do not have sufficient inhibitory activity to potentiate their partner penicillins against derepressed organisms. In combinations of LK-157 with constant concentration ratio (2:1) MICs were evidently reduced in combinations with ceftriaxone and cefotaxime, and were clearly superior to Tazocin^{®}. However, no effect was observed with ceftazidime in the tested strains.

Strain *Klebsiella oxytoca* ATCC 51983 producing ceftazidime resistant broad spectrum β-lactamase (MIC 32-64 mg/L) was susceptible to ceftriaxone and cefotaxime (MIC 2-8 mg/L). In combinations of LK-157 with all cephalosporins MICs were considerably lowered to the range from 0.25 to 2 mg/L.

Strain *Klebsiella pneumoniae* ATCC 700603 producing SHV-18 was resistant to ceftazidime, but susceptible to ceftriaxone and cefotaxime. In combinations of LK-157 with cephalosporins MICs were significantly reduced to the range from 1 to 4 mg/L.

Strain *Klebsiella pneumoniae* ATCC 51503 producing SHV-10 (IRT) and SHV-12 was resistant to all cephalosporins. In combinations of LK-157 with cephalosporins of 3^{rd} generation MICs were significantly reduced, except in combination with ceftazidime. MIC of cefotaxime was lowered to 0.5 mg/L in the combination with LK-157.

All tested Clinical isolates of *Klebsiella pneumoniae* producing ESBLs (group 2be β-lactamases; n=8) were recognized as resistant to ceftazidime. ESBLs are generally inhibited by β-lactamase inhibitors potassium clavulanate, sulbactam and tazobactam, but in our study most of tested *Klebsiella pneumoniae* remained resistant, which can be explained with production of multiple enzymes, inhibitor-resistant TEM β-lactamase (IRT) or noun-β-lactamase mechanism (porin loss). One of the major problems with cephalosporins and ESBL producers is the inoculum effect. Isolates with exceptionally large or small amounts of enzyme may behave anomalously, especially with β-lactamase inhibitor combinations.

There were 3 strains that remained resistant to all tested combinations. However, MIC₅₀s were evidently reduced in combinations with all cephalosporins and the effect was again most pronounced with ceftriaxone and cefotaxime against 6 isolates of *Klebsiella pneumonia.* Their MICs were clearly superior to Tazocin^{®}.

MICs of cephalosporins in combinations with LK-157 against *Citrobacter freundii* B318 producing CTX-M type ESBL were considerably lowered to the range from 1 to 2 mg/L and were superior to Tazocin^{®}.

3 Clinical isolates of AmpC producing *Citrobacter freundii* were recognized as susceptible to 3^{rd} generation cephalosporins and there was no additional lowering of MICs observed in combinations with LK-157. MIC values of 4 resistant *Citrobacter freundii* strains were also considerably reduced. Two strains remained resistant, while the other two were rendered susceptible.

### EXAMPLE 3

Synergistic Effect of β-lactamase Inhibitors When Tested Against Class A β-lactamase Positive Bacterial Strains

Several synergism tests of representative compound of Formula (I) with fixed concentration (4 µg/mL) in combination with amoxicillin, ceftazidime, piperacillin, cefotaxime, ceftriaxone, cefepime, cefpirome and aztreonam were performed (Table 3) and compared with the commercially available β-lactamase inhibitors clavulanic acid, tazobactam and sulbactam.

### Antibiotics

Stock solutions of the test compounds and control antibiotics were prepared in distilled water according to the NCCLS guidelines [Methods for dilution antimicrobial tests for bacteria that grow aerobically. NCCLS document M7-A5; 2000; vol. 19. National Committee for Clinical Laboratory Standards, Villanova, Pa.]. All drug weights were corrected for salt forms and refer to the pure drug substance. Compounds with low water solubility were first dissolved in DMSO (Dimethyl sulfoxide, 20%) and further diluted with water or Mueller Hinton Bouillon (MHB).

### Bacterial strains

All tested strains and Clinical isolates were either purchased from the American Type Culture Collection (ATCC), or from the in-house company culture collection.

### Media

Stock cultures were prepared from broth cultures grown at 35°C for 18-22 h without agitation and subsequent addition of 5 % DMSO (v/v, final concentration) as cryoprotectant and stored frozen in liquid nitrogen. Mueller-Hinton II broth (MHBII)(BBL Cockeysville, Md.) was used for the testing procedure.

### Assay Procedure

The *in vitro* activities of the antibiotics were determined by the broth microdilution method and agar dilution technique as recommended by CLSI guidelines. The MIC experiments were performed in duplicate in 96-well microtiter plates using MHB. Serial twofold dilutions of each antibiotic either alone or in combination with a constant amount (4 µg/mL) of a β-lactamase inhibitor were prepared in MHBII broth. A sterile pipette tip was used to mix the contents of each well (one tip was used going from the low to the high concentration in the same column). The bacterial suspension with final inoculum 10⁵ CFU/mL was transferred to the test medium containing the antibacterial substances by a multipoint inoculator (Dynatech, Chantilly, Virginia, USA). The plates were then incubated at 35°C for 24 h.

Representative compound of Formula (I) was evaluated against the serine-based Class A β-lactamases including TEM-type, SHV-type extended spectrum β-lactamases (ESBL) and various non-ESBL enzymes of enterobacteriaceae as well as BRO-type enzymes of *Moraxella catarrhalis* and penicillinases of *Staphylococcus aureus.*

**Table 3. Synergy of representative compound of Formula (I), clavulanic acid (CLA), sulbactam (SUL) and tazobactam (TZB) with a constant concentration (4 µg/mL) in combination with ceftazidime (CAZ) or piperacillin (PIP) against Class A β-lactamase producing bacteria.**

| **Microorganism** | **β-lactamase** | **CAZ** | | | | | **PIP** | | |
|---|---|---|---|---|---|---|---|---|---|
| | | **alone** | **CLA** | **SUL** | **TZB** | **LK-157** | **alone** | **TZB** | **LK-157** |
| *E coli* | SHV-3 | 1.6 | 01 | 0.1 | 0.1 | 0.1 | >256 | 16 | 1.6 |
| *C freundₗₗ* | SHV-5 | >25.6 | 1.6 | 3.2 | 0.8 | 0.8 | > 25 6 | 3,2 | 3.2 |
| *E cloacae* | SHV-12 | >25.6 | 3.2 | 3.2 | 0.4 | 0 8 | > 25 6 | 3.2 | 12,8 |
| *K pneumoniae* | SHV | >25.6 | > 25.6 | > 25 6 | > 25 6 | 3.2 | - | - | - |
| *K pneumoniae* | SHV | 12.8 | 1.6 | 0.8 | 0.8 | 04 | - | - | - |
| *E coli* | TEM-1 | 0.2 | 0.4 | 0.2 | 0.2 | 0 2 | >256 | 08 | 25.6 |
| *E coli* | TEM-1 | 3.2 | 0.4 | 0 8 | 0.4 | 0 4 | - | - | - |
| *E coli* | TEM-3 | >25 6 | 0.4 | 0.4 | 0.4 | 0 8 | > 25 6 | 1.6 | 1.6 |
| *M catarrhalis* | BRO-1 | 0 2 | 0.1 | 0 1 | 005 | ≤0 0125 | - | - | - |
| *M catarrhalis* | BRO-2 | 0 05 | ≤ 0.0125 | ≤ 0.0125 | ≤ 0 0125 | ≤ 0.0125 | 1.6 | ≤ 00125 | ≤ 0.0126 |
| *S aureus* | PC | 12.8 | 12.8 | 6.4 | 6.4 | ≤ 0 0126 | - | 08 | ≤ 00127 |
| *S aureus* | PC | 25.6 | 12.8 | 6.4 | 12.8 | ≤ 0.0127 | - | - | - |
| *E coli* | ESBL | 1.6 | 0.8 | 0.2 | 0.4 | 0.2 | 0.8 | 0.8 | 1.6 |
| *E coli* | ESBL | 0 4 | 0.1 | 0.1 | 0 2 | 0.2 | - | - | - |
| *E coli* | ESBL | 0.2 | 01 | 0.1 | 01 | 01 | - | - | - |
| *E coli* | ESBL | >25.6 | > 25.6 | 25.6 | 25.6 | 6.4 | - | - | - |
| *C freundₗₗ* | ESBL | >25.6 | > 25.6 | > 25.6 | 25.6 | 1.6 | - | - | - |
| *K oxytoca* | ESBL | 0.4 | 0 1 | 0.1 | 0 2 | 0.2 | 3.2 | 1.6 | 3.2 |

Overall LK-157 expressed similar spectrum of activity as tazobactam in combination with CAZ and PIP against the panel of Class A β-lactamase producing strains. Their MICs were significantly reduced as compared to β-lactam antibiotic alone. Their MICs in combination with CAZ were evidently lower than in combination with PIP, which is partially due to better activities of the antibiotic alone against the tested strains.

Against TEM- and SHV-type ESBLs and other non-characterized ESBLs from Enterobacteriaceae expressing strains the *in vitro* activity of LK-157 combined with CAZ was in the same range as CAZ/clavulanic acid and CAZ/tazobactam.

LK-157 was more active than the other tested β-lactamase inhibitors against BRO-1 and BRO-2 β-lactamases of *M. catarrhalis* and against penicillinases (PC-type) of *S*. *aureus* (MSSA).

### EXAMPLE 4

Synergistic Effect of β-Lactamase Inhibitors When Tested Against Class C β-Lactamase Positive Bacterial Strains

Following procedures identical to those described in Example 3, representative compound of Formula (I) was tested for its ability to enhance antibiotic efficacy against Class C β-lactamase positive bacterial strains that are highly resistant to β-lactam antibiotics. Class C enzymes inCluded AmpC β-lactamases of *Enterobacter Cloacae* and *Citrobacter freundii.* Representative results are presented in Table 4.

**Table 4. Synergy of representative compound of Formula (I) and tazobactam (TZB) with a constant concentration (4 µg/mL) in combination with cefotaxime, ceftriaxone, cefepime, cefpirome, ceftazidime and aztreonam against Class A and C β-lactamase producing bacteria.**

| | **β-lactamase** | | **Cefotaxime** | | | **Ceftriaxone** | | |
|---|---|---|---|---|---|---|---|---|
| **Microorganism** | **type** | **class** | **alone** | **+ TZB** | **+ LK-157** | **alone** | **+ TZB** | **+ LK-157** |
| *E.coli* | TEM-1 | A | >256 | 04 | 04 | >25.6 | 02 | 1.6 |
| *E coli* | TEM-2 | A | 0.1 | 005 | 005 | ≤0.025 | ≤0.025 | ≤0.025 |
| *K pneumomiae* | SHV-18 | A | 32 | 04 | 04 | 64 | 04 | 02 |
| *E coli* | SHV-3 | A | 32 | ≤ 0.025 | ≤ 0 025 | 32 | ≤0.025 | ≤0.025 |
| *C freundₗₗ* | SHV-5 | A | 256 | 005 | 005 | 25.6 | ≤ 0.025 | ≤ 0.025 |
| *E coli* | SHV-5 | A | > 25 6 | > 25.6 | 0.1 | > 25.6 | > 25.6 | 04 |
| *C freundₗₗ* | AmpC | C | > 25.6 | 128 | 32 | > 25.6 | > 25.6 | 32 |
| *E cloacae* | AmpC | C | >25.6 | >25.6 | 128 | > 25.6 | > 25.6 | 256 |
| *E cloacae* | AmpC | C | >25.6 | >25.6 | 32 | > 25.6 | > 25.6 | 32 |
| *E. cloacae* | AmpC | C | >25.6 | 256 | 32 | > 25.6 | 256 | 64 |
| | | | | | | | | |

| | **β-lactamase** | | **Cefepime** | | | **Cefpirome** | | |
|---|---|---|---|---|---|---|---|---|
| **Microorganism** | **type** | **class** | **alone** | **+ TZB** | **+ LK-157** | **alone** | **+ TZB** | **+ LK-157** |
| *E coli* | TEM-1 | A | 12.8 | 0.2 | 0.2 | 25.6 | 0.2 | 0.2 |
| *E coli* | TEM-2 | A | ≤ 0.025 | ≤ 0.025 | ≤ 0.025 | ≤ 0.025 | ≤ 0.025 | 0.05 |
| *K pneumoniae* | SHV-18 | A | 0.8 | 0.2 | 0.2 | 0.8 | 0.2 | 0.2 |
| *E coli* | SHV-3 | A | 0.4 | 005 | ≤ 0.025 | 0.8 | ≤ 0.025 | ≤ 0.025 |
| *E coli* | SHV-5 | A | 25.6 | 3.2 | 0.1 | > 25.6 | 6.4 | 0.1 |
| *C freundₗₗ* | AmpC | C | 0.8 | 0.1 | 0 05 | 1 6 | 0.2 | 0.1 |
| *E.cloacae* | AmpC | C | 0.8 | 0.2 | 0.1 | 6.4 | 1.6 | 0.4 |
| *E cloacae* | AmpC | C | 1.6 | 0.2 | 0.1 | 3 2 | 3 2 | 0.1 |
| *E cloacae* | AmpC | C | 1.6 | 0.2 | 0.2 | 3.2 | 0.8 | 0.2 |
| | | | | | | | | |

| **Microorganism** | **β-lactamase** | | **Ceftazidime** | | | **Aztreonam** | | |
|---|---|---|---|---|---|---|---|---|
| | **type** | **class** | **alone** | **+ TZB** | **+ LK-157** | **alone** | **+ TZB** | **+ LK-157** |
| *E. coli* | TEM-1 | A | 32 | 08 | 08 | 128 | 02 | 02 |
| *E. coli* | TEM-2 | A | 1.6 | 0.1 | 02 | 01 | 0.05 | 005 |
| *K pneumoniae* | SHV-18 | A | 25.6 | 0.8 | 0.8 | > 25.6 | 04 | 04 |
| *E coli* | SHV-3 | A | 08 | ≤0.025 | 005 | 04 | 02 | ≤ 0.025 |
| *E coli* | SHV-5 | A | > 25.6 | >256 | 1.6 | > 25.6 | > 25.6 | 32 |
| *C freundₗₗ* | AmpC | C | > 25.6 | 128 | 1.6 | >256 | 256 | 64 |
| *E cloacae* | AmpC | C | > 25.6 | 128 | 32 | 256 | 128 | 128 |
| *E. cloacae* | AmpC | C | > 25.6 | 25.6 | 3.2 | >256 | 128 | 32 |
| *E cloacae* | AmpC | C | > 25.6 | 128 | 32 | 128 | 256 | 64 |

Representative compound of Formula (I) and tazobactam (TZB) with a constant concentration (4 µg/mL) in combination with cefotaxime, ceftriaxone, cefepime, cefpirome, ceftazidime and aztreonam were used against Class A and C β-lactamase producing bacteria.

Overall LK-157 expressed similar spectrum of activity as tazobactam in combination with β-lactam antibiotics against Class A β-lactamase producing strains. Its activity was superior to tazobactam against Class C β-lactamase (AmpC) producing strains in all combinations used. In some resistant strains the diminished activity of partner β-lactam antibiotic was restored.

The lowest MICs were observed in combination of LK-157 with cefepime and cefpirome.

### EXAMPLE 5

Following procedures identical to those described in Example 2, representative compound of Formula (I) was tested as single agent as well as in combination with either cefotaxime, ceftazidime or cefepime for its ability to enhance antibiotic efficacy against characterized class A, class C and class D β-lactamase positive bacterial strains that are highly resistant to β-lactam antibiotics. Their MICs were compared with that of cefotaxime, cefotaxime/tazobactam, ceftazidime, ceftazidime/tazobactam, cefepime, cefepime/tazobactam, and piperacillin/tazobactam.

Representative results are presented in Table 5, 6 and 7, respectively.

**Table 5. Reduced MIC values (µg/mL) of representative compound of Formula (I) with a constant concentration (4 µg/mL) in combination with cefepime against characterised class A, C and D β-lactamase producing strains.**

| **genus** | **species** | phenotype | functional group* | cefepime | cefepime/ LK-157 | LK-157 | piperacillin/ tazobactam |
|---|---|---|---|---|---|---|---|
| Escherichia | coli | CMY-2 | 1 | 0.5 | 0.25 | 64 | 16 |
| Klebsiella | pneumoniae | CMY-2 | 1 | 0.25 | 0.125 | 128 | 16 |
| Escherichia | coli | CMY-7 | 1 | 2 | 0,5 | 128 | 32 |
| Escherichia | coli | FOX-1 | 1 | 0.25 | 0,25 | 32 | 2 |
| Escherichia | coli | FOX-2 | 1 | 1 | 2 | 128 | 64 |
| Klebsiella | pneumoniae | LAT-1 | 1 | 0.125 | 0.125 | 64 | 8 |
| Klebsiella | pneumoniae | LAT-2 | 1 | 0.5 | 1 | 128 | 128 |
| Salmonella | Spp | DHA-1 | 1 | 1 | 0.5 | 64 | 128 |
| Escherichia | coli | HMS-1 | 2b | 0.125 | 0.125 | 128 | 4 |
| Klebsiella | oxytoca | LXA-1 | 2b | 0,125 | 0,125 | 32 | 2 |
| Escherichia | coli | SHV-1 | 2b | 0,25 | 0.25 | 128 | 4 |
| Escherichia | coli | SHV-2 | 2be | 2 | 0,063 | 64 | 1 |
| Escherichia | coli | SHV-2A | 2be | 16 | 0.125 | 64 | 4 |
| Escherichia | coli | SHV-3 | 2be | 4 | 0,063 | 32 | 2 |
| Escherichia | coli | SHV-4 | 2be | 8 | 0,063 | 32 | 2 |
| Escherichia | coli | TEM-1 | 2b | 0 125 | 0,125 | 128 | 2 |
| Klebsiella | pneumoniae | TEM-1 hyperproduced | 2b | 0,5 | 0,5 | 128 | 128 |
| Escherichia | coli | TEM-2 | 2b | 0,25 | 0,25 | 128 | 256 |
| Escherichia | coli | TEM-3 | 2be | 4 | 0,125 | 128 | 4 |
| Escherichia | coli | TEM-5 | 2be | 0,5 | 0,031 | 32 | 1 |
| Escherichia | coli | TEM-6 | 2be | 4 | 0,125 | 64 | 2 |
| Escherichia | coli | TEM-7 | 2be | 16 | 0,25 | 16 | 1 |
| Escherichia | coli | TEM-8 | 2be | 16 | 0,25 | 128 | 4 |
| Escherichia | coli | TEM-9 | 2be | 128 | 16 | 128 | 4 |
| Klebsiella | pneumoniae | TEM-10 | 2be | 32 | 0,25 | 64 | 4 |
| Escherichia | coli | TEM-20 | 2be | 4 | 0.5 | 64 | 2 |
| Escherichia | coli | TEM-21 | 2be | 4 | 0,125 | 64 | 4 |
| Escherichia | coli | TEM-30 | 2br | 0031 | 0,031 | 64 | 1 |
| Klebsiella | pneumoniae | TEM-30 | 2br | 0,125 | 0,125 | 32 | 32 |
| Escherichia | coli | TEM-31 | 2br | 0,125 | 0.125 | 64 | 8 |
| Klebsiella | pneumoniae, | TEM-31 | 2br | 0,125 | 0,125 | 64 | 8 |
| Escherichia | coli | TEM-32 | 2br | 0.125 | 0,125 | 32 | 128 |
| Escherichia | coli | TEM-34 | 2br | 0.063 | 0,063 | 64 | 2 |
| Escherichia | coli | TEM-35 | 2br | 0.125 | 0,125 | 128 | 16 |
| Escherichia | coli | TEM-36 | 2br | 0,5 | 0.5 | 128 | 16 |
| Escherichia | coli | TEM-50 | 2be | 32 | 4 | 128 | 32 |
| Proteus | mirabilis | TEM-52 | 2be | 16 | 0,25 | 128 | 2 |
| Klebsiella | pneumoniae | TEM-1, SHV | 2b,unknown | 4 | 1 | 128 | 8 |
| Klebsiella | pneumoniae | TEM-1, SHV-5 | 2b, 2e | 16 | 2 | 32 | 128 |
| Klebsiella | oxytoca | K1 highperproduced | 2be | 1 | 1 | 128 | 256 |
| Klebsiella | oxytoca | K1 highperproduced | 2be | 4 | 2 | 128 | 512 |
| Escherichia | coli | OXA-1 | 2d | 0.5 | 0,125 | 64 | 32 |
| Escherichia | coli | OXA-2 | 2d | 0.125 | 0,063 | 128 | 2 |
| Escherichia | coli | OXA-3 | 2d | 0,125 | 0,125 | 128 | 2 |
| Escherichia | coli | OXA-4 | 2d | 0,5 | 0.125 | 64 | 8 |
| Escherichia | coli | OXA-5 | 2d | 0 125 | 0,125 | 64 | 4 |
| Pseudomonas | aeruginosa | OXA-6 | 2d | 8 | 4 | 128 | 32 |
| Escherichia | coli | OXA-7 | 2d | 0,25 | 0,063 | 64 | 32 |
| Pseudomonas | aeruginosa | OXA-10 | 2d | 64 | 8 | 128 | 64 |
| Aeromonas | hydrophila | AER-1 | 2c | 0,5 | 0.125 | 16 | 2 |
| Pseudomonas | aeruginosa | NPS-1 | 2a | 8 | 4 | 128 | 128 |
| Pseudomonas | aeruginosa | PSE-1 | 2c | 32 | 8 | 128 | 64 |
| Pseudomonas | aeruginosa | PSE-3 | 2c | 8 | 4 | 128 | 8 |
| Pseudomonas | aeruqinosa | CARB-3 | 2c | 16 | 16 | 128 | 64 |
| Pseudomonas | aeruginosa | LCR-1 | 2d | 128 | 64 | 128 | 128 |

**Table 6. Reduced MIC values (µg/mL) of representative compound of Formula (I) with a constant concentration (4 µg/mL) in combination with cefotaxime against characterised class A, C and D β-lactamase producing strains.**

| **genus** | **species** | **phenotype** | **functional group*** | **cefotaxime** | **cefotaxime/ LK-157** | **piperacillin/ tazobactam** |
|---|---|---|---|---|---|---|
| Escherichia | coli | CMY-2 | 1 | 32 | 8 | 16 |
| Klebsiella | pneumoniae | CMY-2 | 1 | 16 | 2 | 16 |
| Escherichia | coli | CMY-7 | 1 | 32 | 16 | 32 |
| Escherichia | coli | FOX-1 | 1 | 2 | 2 | 2 |
| Eschenchia | coli | FOX-2 | 1 | 64 | 32 | 64 |
| Klebsiella | pneumoniae | LAT-1 | 1 | 4 | 0,125 | 8 |
| Klebsiella | pneumoniae | LAT-2 | 1 | 16 | 16 | 128 |
| Salmonella | spp. | DHA-1 | 1 | 32 | 8 | 128 |
| Escherichia | coli | HMS-1 | 2b | 0,063 | 0,125 | 4 |
| Klebsiella | oxytoca | LXA-1 | 2b | 0,125 | 0,063 | 2 |
| Escherichia | coli | SHV-1 | 2b | 0,063 | 0,063 | 4 |
| Escherichia | coli | SHV-2 | 2be | 4 | 0,063 | 1 |
| Escherichia | coli | SHV-2A | 2be | 8 | 0,063 | 4 |
| Escherichia | coli | SHV-3 | 2be | 32 | 0,063 | 2 |
| Escherichia | coli | SHV-4 | 2be | 16 | 0,063 | 2 |
| Escherichia | coli | TEM-1 | 2b | 0,063 | 0,063 | 2 |
| Klebsiella | pneumoniae | TEM-1 hyperproduced | 2b | 0,063 | 0,063 | 128 |
| Escherichia | coli | TEM-2 | 2b | 0,063 | 0,125 | 256 |
| Eschenchia | coli | TEM-3 | 2be | 16 | 0,125 | 4 |
| Eschenchia | coli | TEM-5 | 2be | 0,063 | 0,031 | 1 |
| Eschenchia | coli | TEM-6 | 2be | 0.5 | 0,063 | 2 |
| Eschenchia | coli | TEM-7 | 2be | 0,25 | 0,063 | 1 |
| Escherichia | coli | TEM-8 | 2be | 4 | 0,25 | 4 |
| Escherichia | coli | TEM-9 | 2be | 8 | 2 | 4 |
| Klebsiella | pneumoniae | TEM-10 | 2be | 0,5 | 0,063 | 4 |
| Escherichia | coli | TEM-20 | 2be | 2 | 0,5 | 2 |
| Eschenchia | coli | TEM-21 | 2be | 32 | 0,125 | 4 |
| Escherichia | coli | TEM-30 | 2br | 0,031 | 0,031 | 1 |
| Klebsiella | pneumoniae | TEM-30 | 2br | 0,031 | 0,031 | 32 |
| Escherichia | coli | TEM-31 | 2br | 0,063 | 0,125 | 8 |
| Klebsiella | pneumoniae | TEM-31 | 2br | 0,063 | 0,063 | 8 |
| Escherichia | coli | TEM-32 | 2br | 0,125 | 0,125 | 128 |
| Escherichia | coli | TEM-34 | 2br | 0,031 | 0,031 | 2 |
| Escherichia | coli | TEM-35 | 2br | 0,031 | 0,031 | 16 |
| Escherichia | coli | TEM-36 | 2br | 0,25 | 0,125 | 16 |
| Escherichia | coli | TEM-50 | 2be | 8 | 2 | 32 |
| Proteus | mirabilis | TEM-52 | 2be | 16 | 0,063 | 2 |
| Klebsiella | pneumoniae | TEM-1, SHV | 2b, unknown | 4 | 0,5 | 8 |
| Klebsiella | pneumoniae | TEM-1, SHV-5 | 2b, 2e | 32 | 2 | 128 |
| Klebsiella | oxytoca | K1 highperproduced | 2be | 1 | 1 | 256 |
| Klebsiella | oxytoca | K1 highperproduced | 2be | 1 | 1 | 512 |
| Escherichia | coli | OXA-1 | 2d | 0,5 | 0,063 | 32 |
| Escherichia | coli | OXA-2 | 2d | 0,063 | 0,031 | 2 |
| Escherichia | coli | OXA-3 | 2d | 0,063 | 0,063 | 2 |
| Escherichia | coli | OXA-4 | 2d | 0,125 | 0,125 | 8 |
| Escherichia | coli | OXA-5 | 2d | 0,25 | 0,125 | 4 |
| Pseudomonas | aeruginosa | OXA-6 | 2d | 16 | 16 | 32 |
| Escherichia | coli | OXA-7 | 2d | 0,25 | 0,063 | 32 |
| Pseudomonas | aeruginosa | OXA-10 | 2d | 64 | 32 | 64 |
| Aeromonas | hydrophila | AER-1 | 2c | 0,125 | 2 | 2 |
| Pseudomonas | aeruginosa | NPS-1 | 2a | 16 | 16 | 128 |
| Pseudomonas | aeruginosa | PSE-1 | 2c | 16 | 16 | 64 |
| Pseudomonas | aeruginosa | PSE-3 | 2c | 16 | 32 | 8 |
| Pseudomonas | aeruginosa | CARB-3 | 2c | 16 | 16 | 64 |
| Pseudomonas | aeruginosa | LCR-1 | 2d | 64 | 128 | 128 |

**Table 7. Reduced MIC values (µg/mL) of representative compound of Formula (I) in combination with ceftazidime with a constant concentration ratio (2:1) against characterised class A, C and D β-lactamase producing strains.**

| **genus** | **species** | **phenotype** | **functional group*** | **ceftazidime** | **ceftazidime/ LK-157** |
|---|---|---|---|---|---|
| Escherichia | coli | CMY-2 | 1 | >128 | 8 |
| Escherichia | coli | CMY-7 | 1 | >128 | 2 |
| Escherichia | coli | FOX-1 | 1 | 8 | 4 |
| Escherichia | coli | FOX-2 | 1 | >128 | 32 |
| Klebsiella | pneumoniae | CMY-2 | 1 | 64 | 8 |
| Klebsiella | pneumoniae | LAT-1 | 1 | 8 | 2 |
| Klebsiella | pneumoniae | LAT-2 | 1 | >128 | 64 |
| Escherichia | coli | HMS-1 | 2b | 0.25 | 0 25 |
| Escherichia | coli | SHV-1 | 2b | 0.25 | 0 25 |
| Aeromonas | hydrophila | AER-1 | 2c | 0.25 | 0.5 |
| Pseudomonas | aeruginosa | PSE-1 | 2c | 2 | 2 |
| Pseudomonas | aeruginosa | PSE-3 | 2c | 4 | 4 |
| Pseudomonas | aeruginosa | CARB-3 | 2c | 2 | 2 |
| Pseudomonas | aeruginosa | LCR-1 | 2d | 4 | 4 |
| Escherichia | coli | SHV-2 | 2be | 2 | 1 |
| Escherichia | coli | SHV-2A | 2be | 4 | 1 |
| Escherichia | coli | SHV-3 | 2be | 2 | 1 |
| Escherichia | coli | SHV-4 | 2be | >128 | 2 |
| Klebsiella | pneumoniae | TEM-1 hyperproduced | 2b | 0.25 | 025 |
| Escherichia | coli | TEM-1 | 2b | 025 | 0.125 |
| Eschenchia | coli | TEM-2 | 2b | 0.5 | 0 5 |
| Eschenchia | coli | TEM-3 | 2be | 32 | 1 |
| Escherichia | coli | TEM-5 | 2be | 4 | 0.5 |
| Escherichia | coli | TEM-6 | 2be | >128 | 2 |
| Escherichia | coli | TEM-7 | 2be | 32 | 4 |
| Escherichia | coli | TEM-8 | 2be | >128 | 4 |
| Escherichia | coli | TEM-9 | 2be | >128 | 16 |
| Klebsiella | pneumoniae | TEM-10 | 2be | >128 | 8 |
| Escherichia | coli | TEM-20 | 2be | 2 | 1 |
| Eschenchia | coli | TEM-21 | 2be | 16 | 2 |
| Klebsiella | pneumoniae | TEM-30 | 2br | 0.125 | 0 125 |
| Escherichia | coli | TEM-30 | 2br | 0.125 | 0 125 |
| Klebsiella | pneumoniae | TEM-31 | 2br | 025 | 0.25 |
| Escherichia | coli | TEM-31 | 2br | 0 25 | 0.25 |
| Escherichia | coli | TEM-32 | 2br | 0 25 | 0.25 |
| Escherichia | coli | TEM-34 | 2br | 0.125 | 0.125 |
| Escherichia | coli | TEM-35 | 2br | 0.25 | 0.25 |
| Escherichia | coli | TEM-36 | 2br | 0 5 | 0 5 |
| Escherichia | coli | TEM-50 | 2be | 8 | 4 |
| Proteus | mirabilis | TEM-52 | 2be | 4 | 0 5 |
| Klebsiella | pneumoniae | TEM-1, SHV | 2b, unknown | 8 | 2 |
| Klebsiella | pneumoniae | TEM-1, SHV-5 | 2b, 2be | >128 | 8 |
| Klebsiella | oxytoca | K1 hyperproduced | 2be | 1 | 0.5 |
| Klebsiella | oxytoca | K1 hyperproduced | 2be | 05 | 025 |
| Escherichia | coli | OXA-1 | 2d | 0.25 | 025 |
| Escherichia | coli | OXA-2 | 2d | 1 | 0.25 |
| Escherichia | coli | OXA-3 | 2d | 0.5 | 0.25 |
| Escherichia | coli | OXA-4 | 2d | 0.5 | 025 |
| Eschenchia | coli | OXA-5 | 2d | 0.5 | 0.5 |
| Pseudomonas | aeruginosa | OXA-6 | 2d | 2 | 2 |
| Escherichia | coli | OXA-7 | 2d | 05 | 025 |
| Pseudomonas | aeruginosa | OXA-10 | 2d | 4 | 4 |
| Klebsiella | pneumoniae | CTX-M1 | 2be | 16 | 16 |
| Enterobacter | cloacae | CTX-M9 | 2be | 0.5 | 0.5 |
| Escherichia | coli | CTX-M9 | 2be | 2 | 1 |
| Klebsiella | pneumoniae | CTX-M14 | 2be | 8 | 8 |
| Klebsiella | pneumoniae | CTX-M15 | 2be | 32 | 16 |
| Escherichia | coli | CTX-M15 | 2be | 32 | 16 |

Cefepime, cefotaxime and ceftazidime combined with LK-157 exhibited good *in vitro* activity against the 55 laboratory strains producing characterized β-lactamases. LK-157 did not display antibiotic activity *per se* against the tested strains. Therefore significant reduction of MICs observed was due to inhibition of the respective β-lactamases. The most pronounced was activity against TEM- and SHV-type enzymes. Results presented above display broad spectrum of activity against class A, C and D β-lactamases Spectrum of activity is clearly superior to Tazocin^{®} as a golden standard.

Numerous references, including patents, patent applications, protocols and various publications, are cited and discussed in the description of this invention. The citation and/or discussion of such references is provided merely to clarify the description of the present invention and is not an admission that any such reference is "prior art" to the invention described herein.

## Claims

1. A pharmaceutical composition comprising an pharmaceutically effective amount of, (4S,8S,9R)-4-methoxy-10-((E)-ethylidene)-11-oxo-1-azatricyclo[7.2.0.0^{3,8}]undec-2-ene-2-carboxylic acid (LK-157) or a salt thereof,
and an antibiotic,
wherein the antibiotic is a β-lactam antibiotic, wherein said β-lactam antibiotic is a cefalosporine selected from the group consisting of ceftazidime, cefotaxime, cefepime and cefpirome.

2. The pharmaceutical composition according to claim 1, wherein the pharmaceutical composition additionally comprises a pharmaceutically acceptable carrier.

3. The pharmaceutical composition according to any of the claims 1 to 2, wherein the pharmaceutical composition additionally comprises a pharmaceutically acceptable excipient.

4. A pharmaceutical composition as claimed within any of the claims 1 to 3 for use in the treatment of an infection in humans or animals caused by a bacterium.

5. Use of pharmaceutical composition as claimed within any of the claims 1 to 3 for the manufacture of a medicament for the treatment of an infection in humans or animals caused by a bacterium.

## Patentansprüche

1. Pharmazeutische Zusammensetzung, umfassend eine pharmazeutisch wirksame Menge an (45,8S,9R)-4-Methoxy-10-((E)-ethyliden)-11-oxo-1-azatricyclo[7.2.0.0^{3,8}]undec-2-en-2-carbonsäure (LK-157), oder einem Salz davon,
und ein Antibiotikum,
wobei das Antibiotikum ein β-Lactam-Antibiotikum ist, wobei dieses β-Lactam-Antibiotikum ein Cefalosporin ist, ausgewählt aus der Gruppe bestehend aus Ceftazidim, Cefotaxim, Cefepim und Cefpirom.

2. Die pharmazeutische Zusammensetzung nach Anspruch 1, wobei die pharmazeutische Zusammensetzung zusätzlich einen pharmazeutisch annehmbaren Träger umfasst.

3. Die pharmazeutische Zusammensetzung nach einem der Ansprüche 1-2, wobei die pharmazeutische Zusammensetzung zusätzlich einen pharmazeutisch annehmbaren Hilfsstoff umfasst.

4. Pharmazeutische Zusammensetzung wie in einem der Ansprüche 1-3 beansprucht, zur Verwendung bei der Behandlung einer Infektion in Menschen oder Tieren, die durch ein Bakterium verursacht ist.

5. Verwendung der pharmazeutischen Zusammensetzung wie in einem der Ansprüche 1-3 beansprucht, zur Herstellung eines Medikaments für die Behandlung einer Infektion in Menschen oder Tieren, die durch ein Bakterium verursacht ist.

## Revendications

1. Composition pharmaceutique comprenant une quantité pharmaceutiquement efficace d'acide (4S,8S,9R)-4-méthoxy-10-((E)-éthylidène-11-oxo-1-azatricyclo[7.2.0.0^{3,8}] undéc-2-ène-2-carboxylique (LK-157) ou d'un sel de celui-ci,
et un antibiotique,
dans laquelle l'antibiotique est un antibiotique β-lactamine, dans laquelle ledit antibiotique β-lactamine est une céphalosporine choisie dans le groupe consistant en la ceftazidime, le céfotaxime, le céfépime et le céfpirome.

2. Composition pharmaceutique selon la revendication 1, dans laquelle la composition pharmaceutique comprend en outre un support pharmaceutiquement acceptable.

3. Composition pharmaceutique selon la revendication 1 ou 2, dans laquelle la composition pharmaceutique comprend en outre un excipient pharmaceutiquement acceptable.

4. Composition pharmaceutique selon l'une quelconque des revendications 1 à 3, pour utilisation dans le traitement d'une infection chez des humains ou des animaux causée par une bactérie.

5. Utilisation d'une composition pharmaceutique selon l'une quelconque des revendications 1 à 3 pour la fabrication d'un médicament pour le traitement d'une infection chez des humains ou des animaux causée par une bactérie.
